# EUROPEAN PATENT SPECIFICATION

(11) **EP 3 423 074 B1**
(45) Date of publication and mention of the grant of the patent: **13.04.2022**
(21) Application number: 17714288.2
(22) Date of filing: 03.03.2017
(51) Int. Cl.: A61K 38/00, A61K 38/16, A61K 38/17, A61K 38/18, A61P 25/00, A61P 25/28

(54) **INHIBITOR OF ASTROCYTE TNF ALPHA FOR USE IN THE TREATMENT OF NEUROLOGICAL DISEASES**
HEMMER VON ASTROCYT-TNF-ALPHA ZUR VERWENDUNG BEI DER BEHANDLUNG VON NERVENERKRANKUNGEN
INHIBITEUR DU TNF ALPHA DES ASTROCYTES POUR UNE UTILISATION DANS LE TRAITEMENT DE MALADIES NEUROLOGIQUES

(30) Priority: 04.03.2016 IT UA20161364
(43) Date of publication of application: 09.01.2019
(73) Proprietor: Cattaneo, Antonino, 00198 Roma (IT)
(72) Inventor: CAPSONI, Simona, 56127 PISA (PI) (IT); CATTANEO, Antonino, 00198 Roma (IT)
(74) Representative: Fiammenghi, Eva
(86) International application number: PCT/IB2017/051246
(87) International publication number: WO 2017/149503

(56) References cited:
- WO-A1-2008/006893
- WO-A2-2012/170452
- FRANCESCA MALERBA ET AL: "Functional Characterization of Human ProNGF and NGF Mutants: Identification of NGF P61SR100E as a "Painless" Lead Investigational Candidate for Therapeutic Applications", PLOS ONE, vol. 10, no. 9, 15 September 2015 (2015-09-15), page e0136425, XP055310595, DOI: 10.1371/journal.pone.0136425
- SIMONA CAPSONI ET AL: "Intranasal "painless" Human Nerve Growth Factors Slows Amyloid Neurodegeneration and Prevents Memory Deficits in App X PS1 Mice", PLOS ONE, vol. 7, no. 5, 30 May 2012 (2012-05-30), page e37555, XP055310608, DOI: 10.1371/journal.pone.0037555

## Description

### Field of the art

The present invention refers to the medical and biological fields. More in detail the present invention refers to a known mutated form of NGF for a new use in the therapeutic and prophylaxis fields.

### State of the art

Neuroinflammation is an aspect of neurodegeneration which, in various neurological diseases, including Alzheimer's disease, has become a target for the development of new therapies (Frank-Cannon et al 2009; Latta et al., 2015). Neuroinflammation is mediated by glial lineage cells (astrocytes and microglia). Among the different mediators of the immune response, and of neuroinflammation, SDF-1alpha (CXCL12) has received great interest; this is a member of the chemokine family CXC and the only physiological ligand of the receptor CXCR4 (Guyon, 2014). In the central nervous system, SDF-1alpha transcripts are mainly expressed in oligodendrocytes, astrocytes and neurons of the cortex, hippocampus and cerebellum (Stumm et al., 2007). Various studies have demonstrated that SDF-1 promotes neurogenesis and plays an important role in processes of synaptic plasticity, since it promotes and regulates the release of glutamate and GABA (Guyon, 2014).

Regarding neurodegeneration, it was demonstrated that SDF-1alpha promotes the remyelination in multiple sclerosis models (Patel et al., 2010) and the recruitment of neuronal stem cells in cerebral ischemia models (Imitola et al., 2004, Wang et al., 2008). In the case of Alzheimer's disease, the levels of SDF-1alpha are diminished in patients (Parachikova and Cotman, 2007, Zhang et al., 2012) and in transgenic mice Tg2576 which express the human form of APP with the Swedish mutation (Parachikova and Cotman, 2007). In non-transgenic mice, the chronic treatment with the antagonist AMD3100 of the receptor CXCR4 causes learning and memory deficits (Parachikova and Cotman, 2007). The pre-treatment with SDF-1alpha in cell cultures protects the neurons from the apoptosis induced by amyloid beta (Raman et al., 2011). In vivo, the administration of SDF-1alpha inhibits the decrease of the length of the dendrites and spines induced by amyloid beta in the hippocampus (Raman et al., 2011). All these studies suggest that an increase of the concentration of SDF-1alpha could be a potential therapeutic agent for treating Alzheimer's disease and other neurodegenerative and neurological pathologies. (SDF-1alpha Patent). The document WO2007/051785 in fact describes the use of SDF 1 or of an agonist thereof, and in particular the use of SDF-1alpha for producing a medication for the treatment and/or prevention of neurological diseases, even if some diseases like Alzheimer's disease are not expressly claimed.

However, SDF-1alpha is a pleiotropic molecule, with numerous contrasting actions over various systems, including the immune system. SDF-1alpha has numerous pleiotropic effects, also at the level of the central nervous system. In particular, SDF-1alpha is a chemoattractant for lymphocytes T and B, monocytes and neutrophils during the immune response to pathogenic agents (Bleul et al., 1996, Petty et al., 2007). For the purpose of a possible therapeutic use of SDF-1alpha and of its receptor CXCR4 in neurological and neurodegenerative pathologies, this action can have damaging effects, attracting cells of inflammatory and immune nature into a neuronal context already subjected to inflammatory stress (neuroinflammation associated with neurodegeneration), as well as other contrasting pleiotropic effects (Nayak et al 2014).

In addition, specifically in the context of the nervous system, SDF-1alpha increases neuronal death, after bonding with the receptor CXCR4, due to an excessive release of glutamate by astrocytes, mediated by the increased release of the cytokine TNF alpha (Bezzi et al., 2001). In the central nervous system, SDF-1alpha, and its receptor CXCR4, are part of a glia-glia and glia-neurons signaling pathway whose excessive activation is the cause of neurotoxicity and neuronal death (Cali and Bezzi 2010). The activation of the astrocyte receptor CXCR4 by its natural ligand SDF-1alpha (or CXCL12) activates a long and branched chain of intracellular and extracellular events (including the release of the pro-inflammatory cytokine TNFalpha and prostaglandins) which lead to the increase of the release of glutamate by astrocytes (Cali and Bezzi 2010). Generally, it can be stated that an increase of the release of glutamate by the astrocytes can be beneficial, or damaging, depending on the level of synaptic glutamatergic transmission. Nevertheless, an increase of the proinflammatory cytokine TNF alpha is certainly damaging, even more so in a neuronal context already affected by neuroinflammation (Habbas et al 2015). Indeed, increased levels of the cytokine TNFalpha cause persistent synaptic alterations in the hippocampus and consequent grave cognitive deficits (Santello and Volterra 2012; Habbas et al 2015).

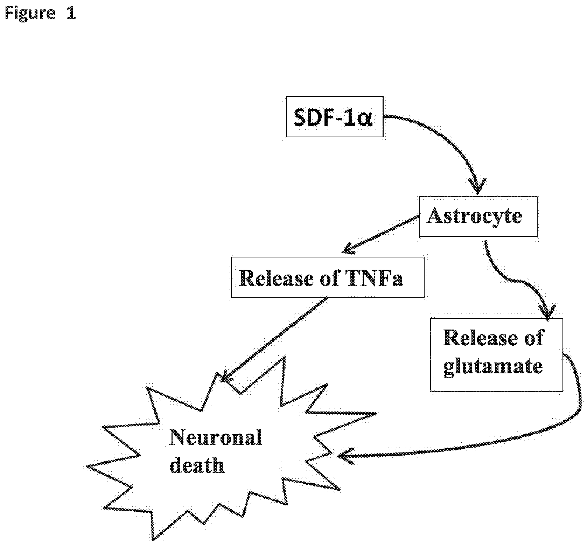

Hence, it can be included that there are two effects induced by the activation of the SDF1-alpha/CXCR4 pathway, with a certainly negative action represented by the induction of the astrocyte TNFalpha (Fig. 1).

For such purpose, the object of the present invention is to provide a local topical activator of the SDF-1alpha pathway, and in particular activators of its expression or of its action, which allow exerting the positive effects of the chemokine SDF-1alpha, but that do not have the side effects thereof such as the induction of chemotaxis of cells of the immune system, nor the induction of an excessive release of glutamate and consequent neuronal death, nor even the induction of the cytokine TNFalpha.

Before commencing the following detailed description, and for the purpose of a clearer comprehension thereof, it should be noted that a mutated form of the human NGF was characterized in a series of preceding studies: hNGFP61S/R100E (NGF painless hNGFp) (Covaceuszach et al 2010: Capsoni et al PlosOne 2011; Capsoni and Cattaneo Patent). The molecule hNGFp is a variant of the molecule of wild type NGF. The protein NGF, even if having neurotrophic properties of therapeutic relevance for various neurodegenerative and neurological pathologies, is negatively affected by the undesired property of having a powerful nociceptive, algogenic and pain sensitization action (Reference Apfel; Pezet and Macmahon 2006;). In order to overcome this problem, which limits the therapeutic actions of the wild type NGF, the molecule hNGFp was developed that is the object of the document WO2008006893: mutations were introduced at the level of two amino acids in the sequence of the mature NGF. The first substitution, from proline 61 to serine, renders the mutated NGF detectable by means of ELISA assays, with regard to the endogenous NGF (Covaceuszach et al., 2009, Malerba et al., 2015). The second mutation is based on the mutation R100W of the NGF gene present in patients affected by sensory and autonomic neuropathy type V (Einarsdottir et al., 2004). These patients are affected by pain insensitivity (Einarsdottir et al., 2004). By introducing the mutation R100E, which has effects similar to the pathogenic mutation in terms of signal transduction, a double mutant hNGFP61S/R100E (hNGF painless, hNGFp) was obtained, characterized by identical neurotrophic properties but also by a capacity to induce pain that is 10 times less than that of wild type NGF (Malerba et al., 2015). With regard to its interactions with the membrane receptors, hNGFp is a selective agonist of the receptor TrkA, while it binds the receptor p75NTR with an affinity 100 times less than the wild type NGF (Covaceuszach et al 2010). It is necessary to underline that according to the prior art, all the neurotrophic properties of wild type NGF are identically preserved by hNGFp, and only the capacity of nociceptive sensitization is 10 times less in NGFp than in wild type NGF. The object of the present invention is therefore to promote the activation of the SDF-1alpha pathway, simultaneously avoiding the side effects associated with this chemokine. This occurs by means of particular administrations of hNGFp.

### Description of the invention

The present description refers to a new use of a known mutated human form of NGF. Said mutated human form will be indicated, in the course of the present description, as hNGFp. More in detail, by hNGFp it is intended a human mutated form of NGF comprising two mutations with respect to the form of the wild type NGF. The main mutation is the aforesaid mutation R100E, which confers to the mutated form the property of not activating the pain sensitization pathways (the indication "p" refers to the "painless" condition), but being equally neurotrophic. The other mutation, P61S, does not at all alter any of the known properties of the wild type NGF; it is therefore "neutral" from the biological standpoint, but renders the molecule hNGFp recognizable by the endogenous molecules of NGF, by means of a specific immunoassay which was suitably developed. More clearly, this allows distinguishing the forms of therapeutic NGF from the endogenous forms. Since the doses of endogenous NGF vary from person to person, and in the same individual in accordance with many factors, stress and approach, the possibility of specifically dosing therapeutic NGF with respect to endogenous NGF is a very advantageous condition.

Still more in detail, the two mutations regard: the first: the substitution of the proline amino acid in position 61, in particular the substitution of the proline 61 with a serine; the second: the substitution of an amino acid in any one of the positions 95-101. By way of a non-limiting example, the substitution of the arginine in position 100. Preferably, said arginine is substituted with tryptophan or with an amino acid selected between glutamic acid and aspartic acid.

More in detail, the present description will refer to the sequences hNGFP61SR100E and hNGFP61SR100W, regarding which the following accession numbers are reported:
BankIt1936238 hNGFP61SR100E KX548900
BankIt1936247 hproNGFP61SR100W KX548901

More specifically, the object of the present industrial invention patent application is that of describing and claiming hNGFp (object of said prior art document (WO2008/006893)) for simultaneous use as agent for the activation of the SDF-1alpha pathway and as agent for the inhibition of the activity of astrocyte TNF alpha. Another object of the present industrial invention patent application is to describe and claim a particular administration path for hNGFp in order to obtain said new technical effect. More specifically, the latter consists of attaining all the benefits deriving from the activation of the pathway of the chemokine SDF-1alpha - a chemokine known for its anti-amyloidogenic and anti-neurodegenerative activity - and from the inhibition of astrocyte TNF alpha whose presence mediates an excessive release of glutamate by the astrocytes, as well as other negative effects. More in detail said technical effect consists of promoting the neurogenesis and anti-amyloidogenic activity and of inhibiting the excessive and normally-encountered side effect of glutamate release. The technical effect is thus seen in the dual action of hNGFp which, as will be described hereinbelow, is unexpected.

More in detail, in the course of the studies pertaining to the present invention, it was found that the painless NGF (hNGFp) is a local activator of the SDF-1alpha pathway, and that through the activation of this pathway it exerts a powerful anti-amyloidogenic and anti-neurodegenerative action. Indeed, by blocking this pathway, with an inhibitor that blocks the interaction between SDF-1alpha and its receptor CXCR4, these positive effects of hNGFp are blocked. Nevertheless, surprisingly, and unexpectedly, it was found that an increased expression of SDF-1alpha by hNGFp does not induce an corresponding increase of the levels, nor of the activity of the proinflammatory cytokine TNFalpha, an event that would be undesired and counter-productive. This is doubly unexpected, since i) SDF-1alpha activates the production of astrocyte TNF-alpha and ii) NGF wild type is a powerful inducer of the expression of TNFalpha (Barouch et al 2001; Takei and Laskey 2008). Hence, the property of not inducing TNF alpha is a new unexpected property of hNGFp, that could not be predicted beforehand, and useful in light of that stated above.

### Detailed description of the invention

The invention will be described in detail hereinbelow with reference to the enclosed figures, in which:
FIGURE 1 shows: a scheme of the negative effects of SDF-1α on astrocytes due to the excessive production of TNFα and glutamate. These effects lead to neuronal death.
FIGURE 2 shows: (A) the immunohistochemistry for amyloid beta Aβ in the cerebral cortex, hippocampus and subiculum of 5xFAD mice after intranasal administration of hNGFp, (B) the quantification of the quantity of amyloid beta plaques in the cerebral cortex, hippocampus and subiculum after intranasal treatment with hNGFP (the bars of the histograms are representative of mean ± standard error, n = 8 per group, one-way analysis of the variance (ANOVA), Bonferroni post-hoc test, P < 0.001).
FIGURE 3 shows: hNGFp promotes the reduction of the pro-amyloidogenic processing of APP. Western blots for (a) PS1, anterior pharynx 1a (APh1) and Pen-2, (b) Nicastrin (NCT), (c) BACE1 (d) Neprilysin (NEP) (e) full length APP (fAPP), (f) c-terminal fragments and (h) insoluble Aβ. The images are representative of 3 independent experiments. (g) Dot blot for verifying the expression of the amyloid beta oligomers (the bars of the histograms are representative of mean ± standard error, one-way analysis of the variance (ANOVA), Bonferroni post-hoc test, P < 0.001).
FIGURE 4 shows: (A) Increase of the cholinergic innervation after intranasal administration of hBDNF. (B) Lack of decrease of the number of amyloid beta plaques after intranasal administration of hBDNF (one-way analysis of the variance (ANOVA), n = 8 per group, Bonferroni post-hoc test, P < 0.001).
FIGURE 5 shows: in the 5xFAD mice, the cortical neurons which produce amyloid beta (in a) do not express the receptor for NGF TrkA. Markers for the neurofilament M and for TrkA.
FIGURE 6 shows: scheme of the intraparenchymal injection site of hNGFp.
FIGURE 7 shows: (A) immunohistochemistry for the choline acetyltransferase (ChAT) in the cholinergic nucleus basalis of Meynert and for amyloid beta Aβ in the cerebral cortex of the 5xFAD mice after intraparenchymal administration of hNGFp. (B) Quantification of the cholinergic fibers and of the quantity of amyloid beta plaques in the cerebral cortex after intraparenchymal treatment with hNGFP (the bars of the histograms are representative of mean ± standard error, n = 4 per group, one-way analysis of the variance (ANOVA), Bonferroni, P < 0.001).

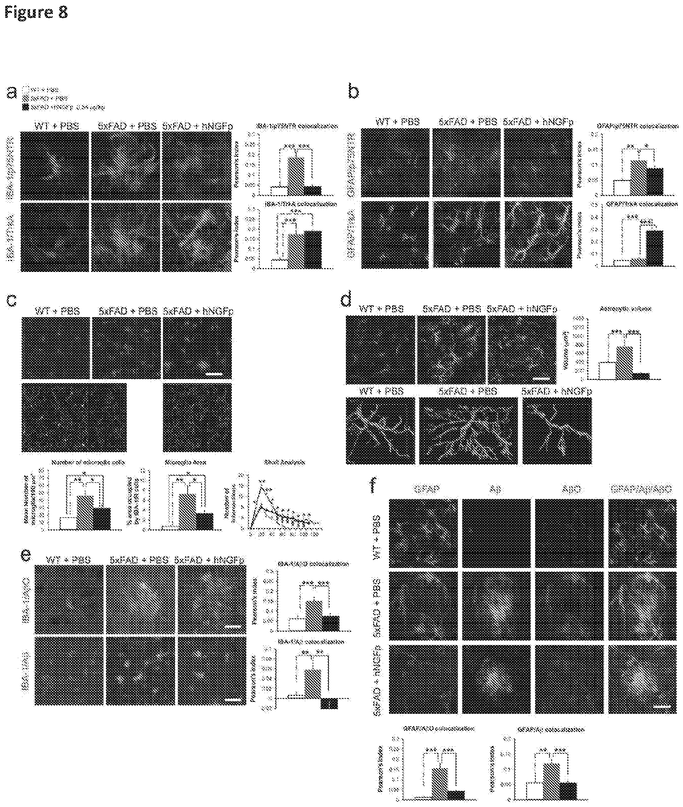

FIGURE 8 shows: (a) Confocal images and quantification of the co-localization of p75NTR and TrkA in the microglia cells (n = 8). (b) Confocal images and quantification of the co-localization of p75NTR and TrkA in the astrocytes (n = 5). (c) Confocal images for showing the microglial marker IBA-1 and quantification of the number of microglial cells, of the area occupied by these cells and of the number of ramifications (non-transgenic mice n = 71 cells; 5xFAD mice treated with PBS n = 99 and 5xFAD mice treated with hNGFp n = 61). (d) Confocal images for showing the astrocyte marker GFAP and quantification of the volume of astrocytes (n = 20 cells per treatment group). (e) Confocal images and co-localization of IBA-1, oligomers of amyloid beta and total amyloid beta in the microglia (n = 7 cells per group). (f) Confocal images and co-localization of GFAP, oligomers of amyloid beta and of total amyloid beta in the astrocytes (n = 7 cells per group) (^{∗} P < 0.05, ^{∗∗} P < 0.001, ^{∗∗∗} P< 0.0001).
FIGURE 9: shows the expression of the receptors for NGF in the cortex of Alzheimer (AD) patients and non-demented patients (CTRL). (A) TrkA and (B) p75 in microglia marked with an antibody against IBA-1. (C) TrkA and (D) P75 in astrocytes marked with an antibody directed against GFAP.
FIGURE 10 shows: increased phagocytosis of the oligomers of amyloid beta by the microglia in the presence of hNGFp and not of hwild type NGF.
FIGURE 11 shows: increased phagocytosis of the amyloid beta oligomers by the astrocytes in the presence of hNGFp.
FIGURE 12 shows: Quantification of the immunoblot used for the analysis of the inflammatory markers. (one-way analysis of the variance (ANOVA), n = 4 per group, Bonferroni post-hoc test, P < 0.05).
FIGURE 13 shows: Confocal images and quantification of the co-localization of amyloid beta, NF-M and SDF-1α in cortical neurons (one-way analysis of the variance (ANOVA), n = 9 per group, Bonferroni post-hoc test, P < 0.0001).
FIGURE 14 shows: (A) Quantification of the percentage of neurons that express SDF-1alpha after treatment with hwild type NGF; (B) Orofacial test for measuring the mechanical allodynia in the region of the nose after administration of increasing doses hNGFp or hwild type NGF (n = 10 per group, one-way analysis of the variance (ANOVA), Bonferroni post-hoc test, ^{∗}P < 0.001).
FIGURE 15 shows: decrease of the expression of SDF-1alpha in cortical neurons after incubation with 50ng/ml of TNFalpha.

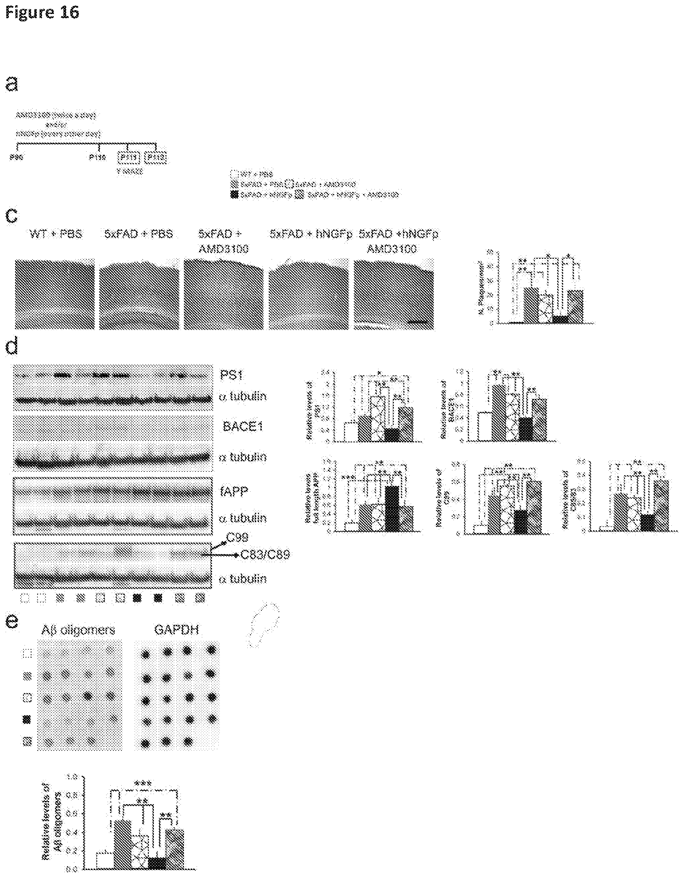

FIGURE 16 shows: the administration of the inhibitor of the receptor CXCR4 of SDF-1alpha prevents the improvement of the neurodegenerative phenotype determined by the intranasal administration of hNGFp (a) Scheme of the treatment (b) Test of the Y maze for evaluating the spatial memory (n = 10; one-way ANOVA, Bonferroni post-hoc test, ^{∗} P < 0.05, ^{∗∗} P < 0.001). (c) Immunohistochemistry for amyloid beta in the cerebral cortex and quantification of the number of plaques (d) Western blots for presenilin 1, BACE 1, full length APP and C-terminal fragments. (e) Dot blot for the quantification of the oligomers of amyloid beta.
FIGURE 17 shows: the co-incubation of hNGFp with the inhibitor AMD3100 does not affect the decrease of oligomers in the microglia (A) and the decrease of non-oligomer Abeta in the astrocytes (B) observed after treatment with hNGFp on its own.
FIGURE 18 shows: (A-C) recovers the neurogenesis in Down syndrome model mice after administration of hNGFp. (D) quantification of the recovery of the neurogenesis deficit.
FIGURE 19 shows: (A) quantification of the volume of astrocytes in Down syndrome model mice after treatment with hNGFp. (B) Western blot for GFAP and (C) quantification of the GFAP level reduction after treatment with hNGFp.
FIGURE 20 shows: (A) after intranasal administration, hNGFp determines the recovery of the memory deficit in recognizing objects; (B) after subcutaneous administration, hNGFp determines the recovery of the sensitivity to painful stimulus of thermal type.

### Experimental data:

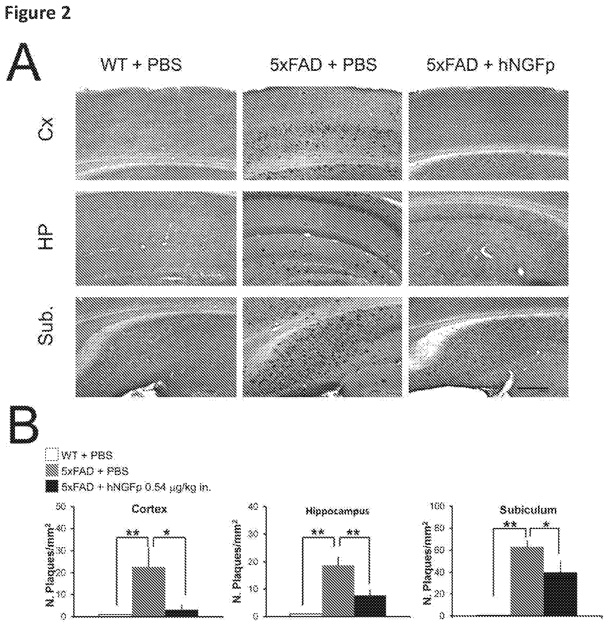

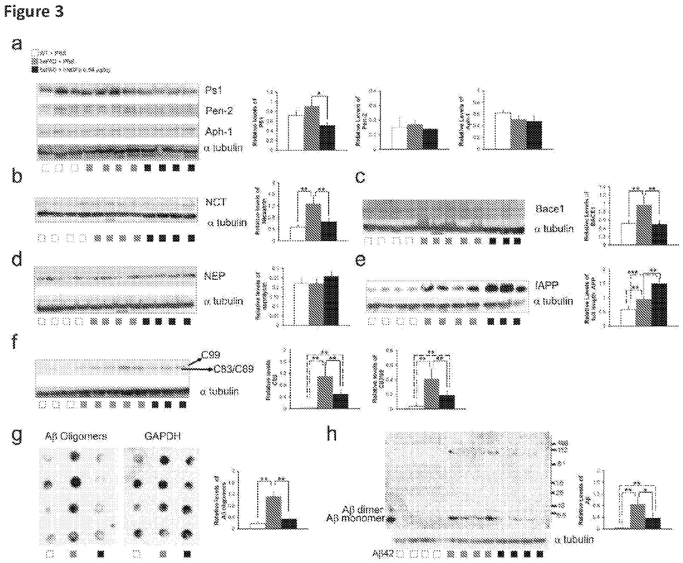

hNGFp was administered intranasally with a very aggressive murine model of Alzheimer's disease, the 5xFAD mouse (Oakley et al., 2006), which has 5 gene mutations corresponding to the same number of mutations that lead to familiar forms of Alzheimer's disease. After intranasal delivery of hNGFp, a powerful anti-amyloidogenic action is observed at the dose of 0.54 µg/kg (Fig. 2), an action characterized by a dramatic reduction of the number of amyloid beta plaques (Fig. 2). The mechanism of this anti-amyloidogenic action of hNGFp is determined by the fact that this neurotrophin diminishes the expression of presenilin 1 (Fig. 3a), nicastrin (Fig. 3b) and BACE1 (Fig. 3), i.e. of proteins of the enzymatic complexes that cut the precursor protein of the amyloid APP, forming the amyloid beta peptide (Fig. 3d, e). The result of this action is a lower production both of oligomers of the amyloid beta peptide (Fig. 3g) and of amyloid beta aggregates (Fig. 3f). The expression of degradation of amyloid beta, neprilysin, is instead not significantly varied (Fig. 3d).

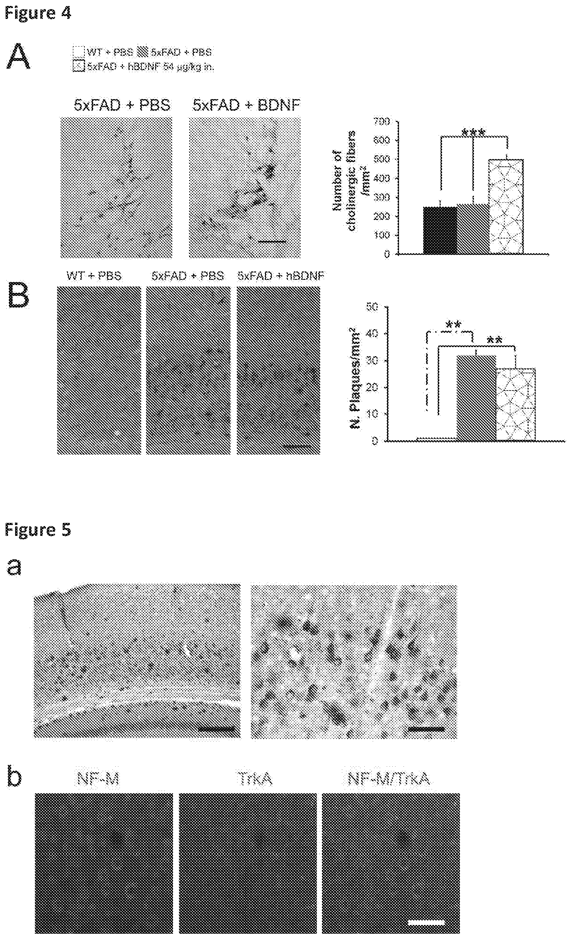

Surprisingly, this action of the hNGFp is specific for this neurotrophin. Indeed, the administration of doses up to 100 times greater of the neurotrophin hBDNF, very similar to NGF (and hence also to hNGFp), determine an increase of the cholinergic innervation (Fig. 4A) but does not decrease the number of amyloid beta plaques (Fig. 4B). Hence, the action of hNGFp is not generically neuroprotective, but involves a specific mechanism that leads to the reduction of the formation of amyloid beta peptide.

This very effective anti-amyloidogenic and anti-neurodegenerative action of hNGFp poses a question: which are the cells of the brain that mediate the actions of hNGFp? In fact, the neurons that produce amyloid beta do not express TrkA (Fig. 5), the main receptor for hNGFp (Covaceuszach et al., 2010). The only cell population in the CNS that is known and validated as target of the NGF action (Rosenberg et al., 1988, Smith et al., 1999, Tuszynski, 2000, Salehi et al., 2006, Nagahara et al., 2009) is constituted by the cholinergic neurons of the basal forebrain, which innervate all the cortical areas in a distributed and diffused manner. Other neuronal populations, or other cell types (microglia and astrocytes) do not express TrkA receptors in the brain in physiological conditions (Table 1).

**Table 1:**

| Modulation of the expression of the receptors p75NTR and TrkA in the microglia and in the astrocytes after treatment with hNGFp. | | | |
|---|---|---|---|
| | **P75NTR** | | |
| **Cell type** | **WT+PBS** | **5xFAD + PBS** | **5xFAD + hNGFp** |
| Microglia | - | ↑ | ↓ |
| Astrocytes | - | ↑ | ↓ |

| | **TRKA** | | |
|---|---|---|---|
| **Cell type** | **WT+PBS** | **5xFAD + PBS** | **5xFAD + hNGFp** |
| Microglia | - | ↑ | ↑ |
| Astrocytes | - | - | ↑ |

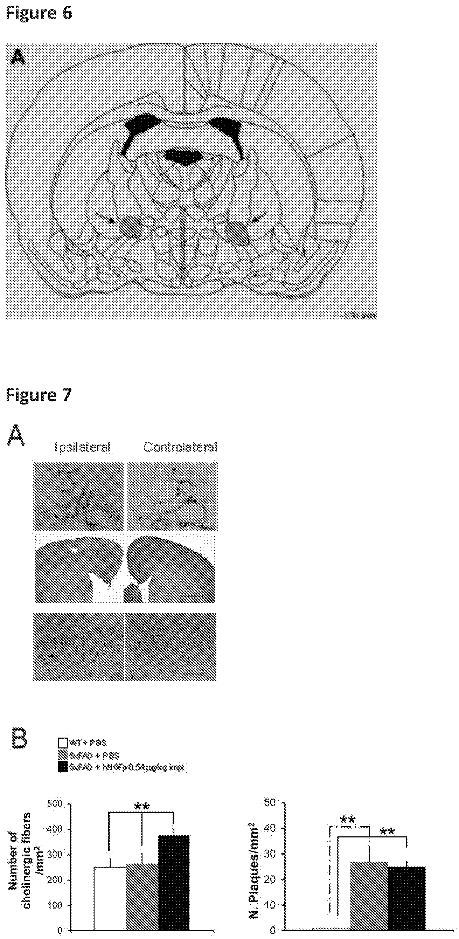

In order to verify if the activation of the cholinergic neurons by the hNGFp is responsible for the observed anti-amyloidogenic effects, we have administered hNGFp via intraparenchymal administration by means of osmotic minipumps, which release the neurotrophin directly in proximity to the cholinergic neurons of the basal forebrain (Fig. 6). The hNGFp was administered at the same dose used intranasally (0.54 µg/kg). We have observed that the hNGFp determines, as expected, the increase of the cholinergic innervation (Fig. 7A,B), but surprisingly does not determine a reduction of the number of amyloid beta plaques (Fig. 7A,B) and results entirely ineffective after this localized administration. It follows that i) the action of the hNGFp requires a diffused biodistribution and that ii) surprisingly and unexpectedly the anti-amyloidogenic action of the hNGFp is not mediated by the cholinergic neurons but rather by the cells of the microglia and by the astrocytes. Indeed, contrary to what occurs in physiological conditions (Table 1), the microglia cells, in neurodegeneration conditions, express both the receptors TrkA and p75NTR (Fig. 8a and Table 1). In the same conditions, the astrocytes express only p75NTR (Fig. 8b and Table 1). Therefore, it can be concluded that in neurodegeneration and neuroinflammation conditions, microglial and astrocyte cells acquire the capacity to respond to hNGFp.

The expression of these receptors is modulated by the hNGFp itself (Table 1): following the treatment with hNGFp, the expression of p75NTR is decreased in both cell types, while the expression of TrkA remains constant in the microglia and increases in the astrocytes.

In these cells, the intranasal administration of hNGFp to 5xFAD mice determines:
(1) in the microglia cells: a decrease of the number, of their area and an increase of the number of ramifications, with respect to that detected in the brains of the control 5xFAD mice, treated with PBS (Fig. 8c). In addition, a reduction is observed of the quantity of immunoreactive cells for the different species of amyloid beta and in particular of oligomers (Fig. 8e).
(2) in the astrocytes: a decrease of the volume of the single cells, signifying a decrease of astrocytosis (Fig. 8d). In addition, also for the astrocytes, a reduction is observed of the quantity of immunoreactive cells for the different species of amyloid beta, and in particular of oligomers (Fig. 8f).

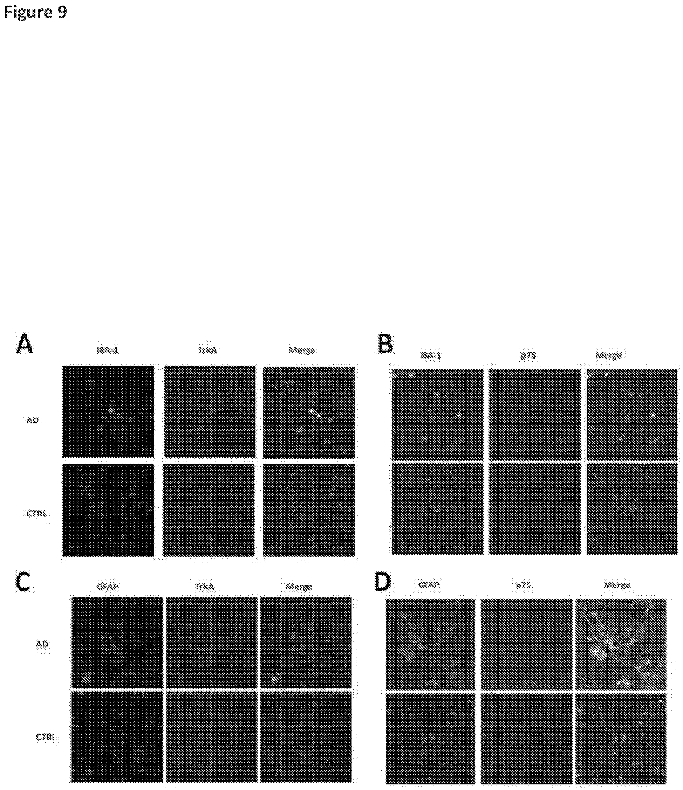

The expression of the receptors for NGF was also verified post-mortem in parietal cortex tissues of Alzheimer patients. In this cerebral area, the expression of the receptors TrkA and p75 results increased in the microglia (Fig. 9A, B) and in the astrocytes (Fig. 9C, D). Therefore, also in the human cerebral tissue affected by Alzheimer's disease, the astrocyte and microglial cells become responsive to NGF, by means of expression of the corresponding receptors (which do not express in physiological conditions in vivo). Such cells therefore become mediators of the specific actions of hNGFp.

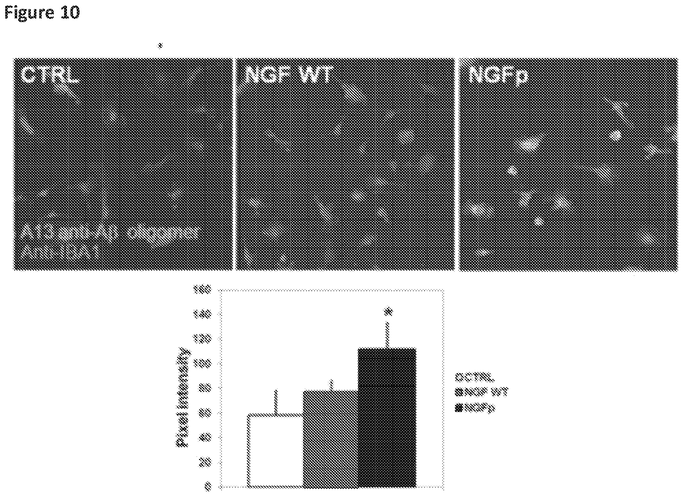

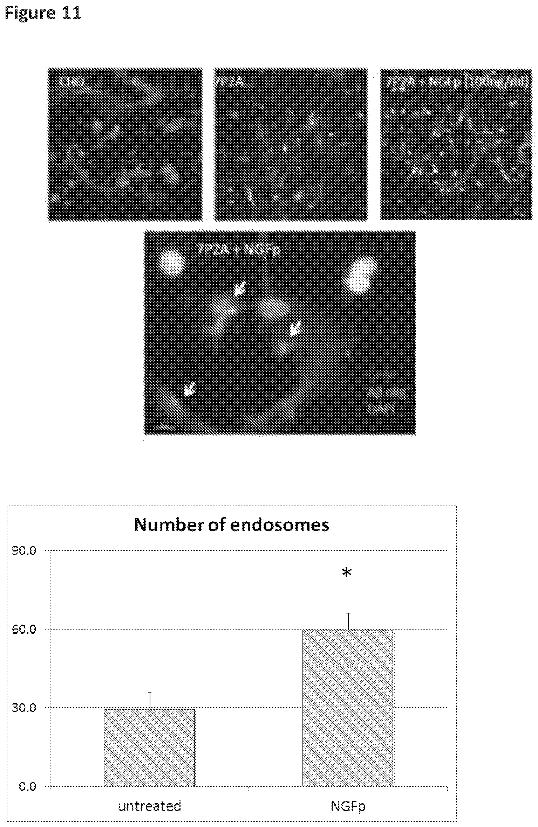

In cultured cells, the treatment with hNGFp determines an increase of the phagocytosis of the oligomers of amyloid beta by cultured microglia cells (Fig. 10) and by cultured astrocytes (Fig 11). In an unexpected manner, and not expected based on the known or predictable properties of hNGFp, hNGFp is more effective than the wild type NGF in inducing phagocytosis of amyloid beta oligomers by the microglia (Fig 10).

These results demonstrate that the treatment with hNGFp determines an increase of the capacity to remove pathological forms of the amyloid beta peptide. The verification of this event poses the question: how can the action of hNGFp on the glial cells also determine the observed reduction of the production of amyloid beta in the neurons (Fig. 2)?

The microglia can be considered the primary cellular target of hNGFp in the brain of the 5xFAD mice, since: (1) hNGFp is bonded nearly exclusively to the receptor TrkA and does not effectively bond the other receptor p75NTR (Covaceuszach et al., 2010); (2) the microglia is the only non-neuronal cell type that expresses TrkA, in the brain of the 5xFAD mice that are not treated or treated with PBS. Since one of the functions of the microglia is that of secreting soluble factors that act in an autocrine and paracrine manner (Nayak et al 2014), it is assumed that a soluble factor can be secreted by the microglia and decrease the production of Abeta.

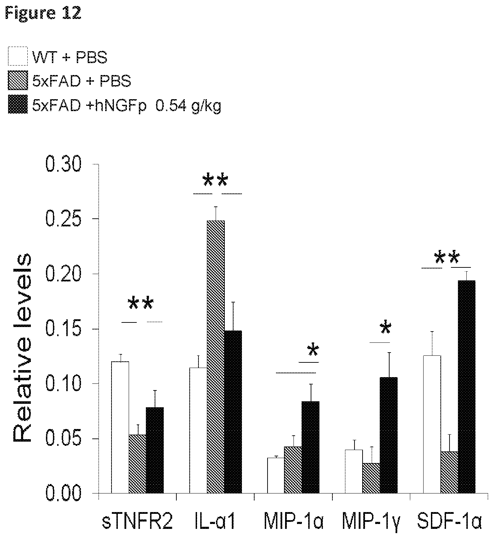

In order to identify this factor, an array of antibodies was used that allows identifying the main cytokines and chemokines modulated during the inflammation states. Among those analyzed, 5 factors are modulated by hNGFp:
(1) The expression of the soluble receptor 2 of TNFalpha (sTNFRII), and of the chemokines MIP-1alpha (CCL3), MIP-1gamma (CCL9) and SDF-1alpha (CxCL12) is increased (Fig. 12 and Table 2).
(2) The expression of the cytokine IL-1alpha is decreased (Fig. 12 and Table 2).

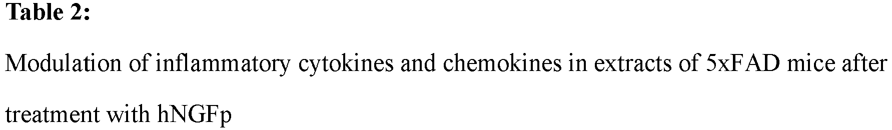

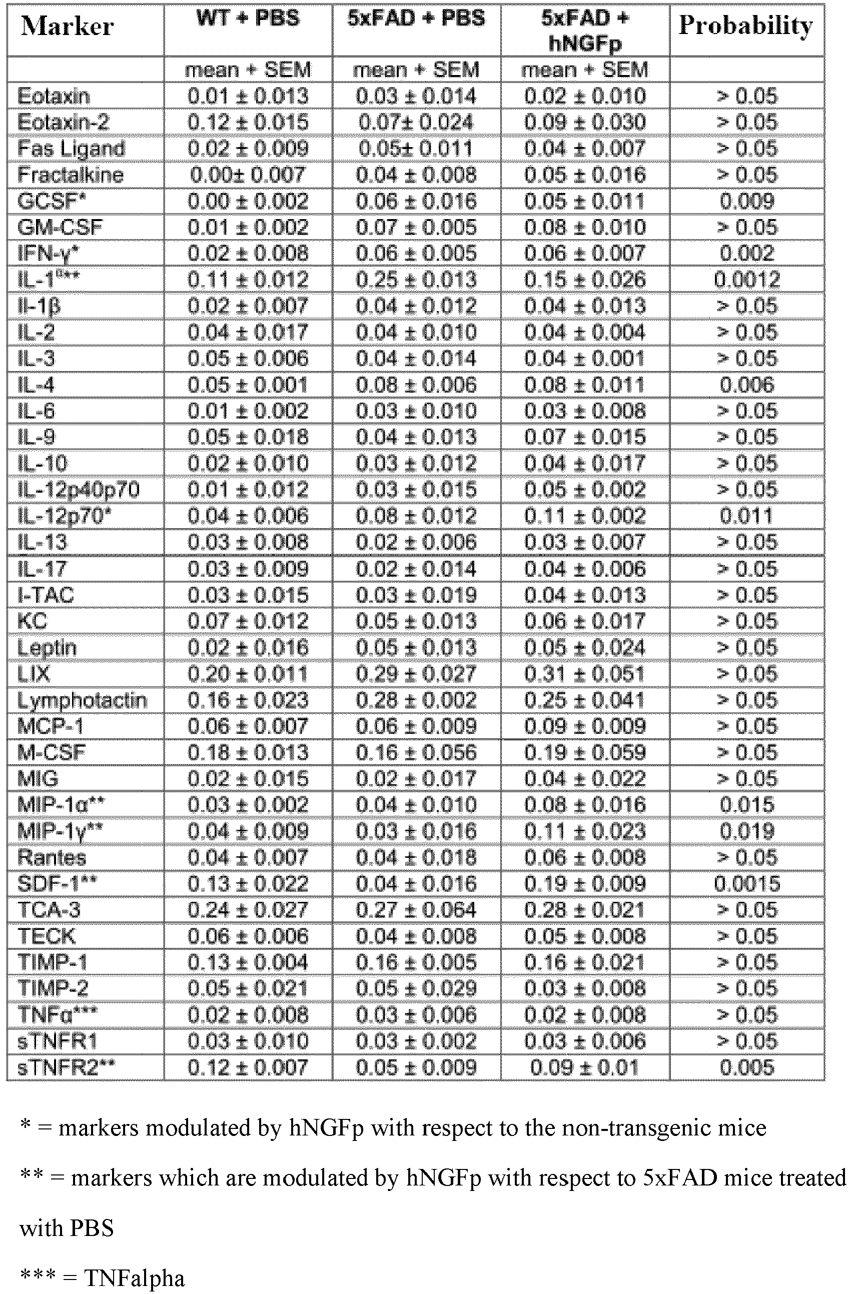

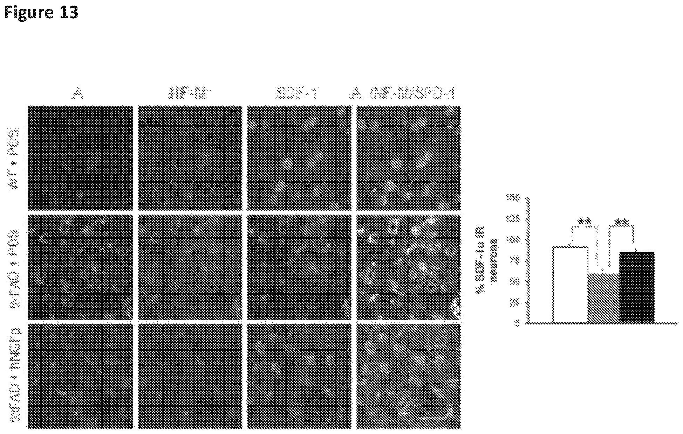

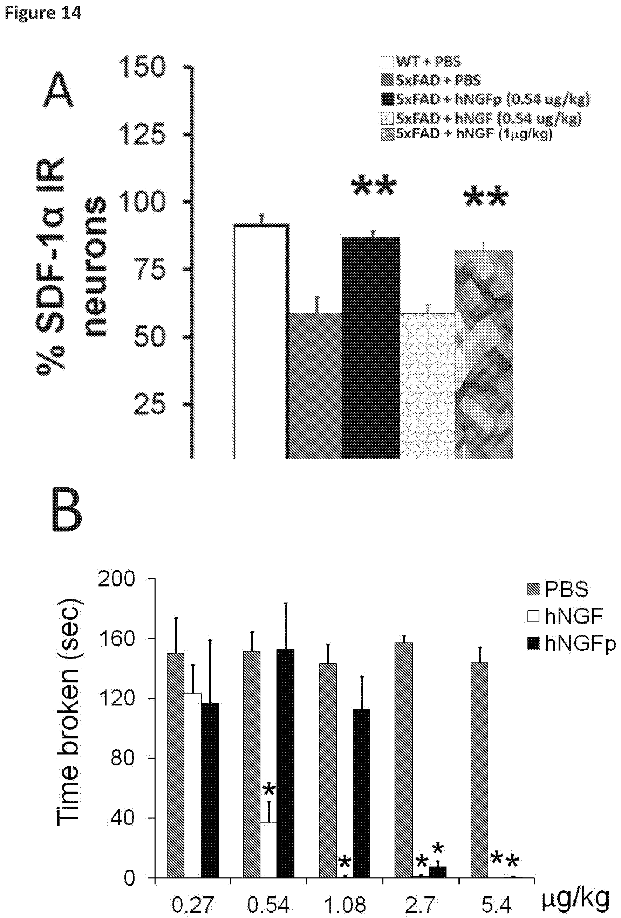

In addition, by means of immunohistochemistry, it is observed that the expression of SDF-1alpha is specifically increased in the neurons that produce amyloid Abeta (Fig. 13). Surprisingly, an analogous treatment with hNGF wildtype at the same dose of hNGFp (0.54 µg/kg) does not determine an increase of SDF-1alpha (Fig. 14A). This induction can only be obtained at very high doses (da 1 µg/Kg), which have as negative effect the activation of the nociceptors of the trigeminal nerve and the consequent induction of pain in the orofacial region (Fig. 14B).

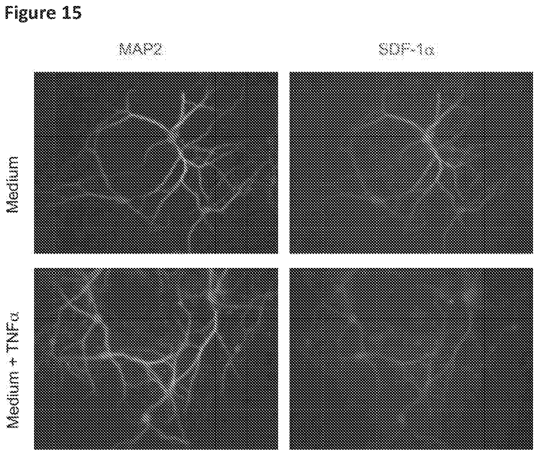

It is particularly significant that among the cytokines that are instead not modulated by hNGFp, there is TNFalpha. This is surprising and unexpected, i) since it is known that the wild type NGF is, on the contrary, a powerful inducer of TNFalpha (Barouch et al 2001; Takei and Laskey 2008), ii) and in light of the fact that hNGFp instead induces SDF-1alpha (Fig, 12 and Table 2), which in turn is a known inducer and activator of TNFalpha (see above). Hence, the property of not inducing TNF alpha is a new unexpected property of hNGFp, not predictable beforehand, and useful in light of that stated above. In addition, the fact that hNGFp instead stimulates the production of the soluble receptor 2 of TNFalpha (sTNFRII) (a known decoy inhibitor of the TNFalpha action) contributes to ensuring that the final result of the treatment with hNGFp is that of reducing the general activity of TNFalpha. Since it is known that in tissues of non-neuronal origin an increase of TNFalpha causes a decrease of the expression of SDF-1alpha (Zhang et al., 2008, Bockstal et al., 2011), the reduced tone of TNFalpha correlates with the observed increase of SDF-1alpha (Fig. 12 and Table 2). It is verified that this relation also existed in cultured neurons, where it is confirmed that TNFalpha reduces the expression of SDF-1alpha (Fig. 15).

Overall, all these results propose the possibility that, surprisingly and unexpectedly, the neuroprotective, anti-amyloidogenic and anti-neurodegenerative actions of hNGFp are mediated by the chemkine SDF-1alpha, without however there being the onset of undesired involvement of the cytokine TNFalpha, which normally is in the cascade activated by SDF-1alpha.

In order to demonstrate this assumption, thus passing from a correlation to a demonstration, in the course of the experiments pertaining to the present invention a known inhibitor of the receptor CXCR4 of the chemokine SDF-1alpha was used, AMD3100. The co-treatment of the 5xFAD mice with hNGFp (0.54 µg/kg) and AMD3100 (1 mg/kg twice per day) completely blocks the anti-amyloidogenic action of hNGFp, since the decrease of the number of amyloid beta plaques is no longer observed (Fig. 16a,c), nor is the reduction observed of the expression of presenilin 1, nicastrin and BACE1 and of the oligomers of Abeta (Fig. 16d,e) which would have been obtained following treatment with hNGFp on its own.

Unexpectedly, not all the parameters measured in the microglia and in the astrocytes and improved after intranasal administration of hNGFp are blocked by the co-administration of AMD3100.

**Table 3:**

| Modulation of the expression of the receptors p75NTR and TrkA in the microglia and in the astrocytes after treatment with hNGFp. | | |
|---|---|---|
| **Cell type** | **hNGFp** | **block after co-treatment with AMD3100** |
| Abeta deposition diminution | + | + |
| APP processing diminution | + | + |
| Diminution of Abeta oligomers in neurons | + | + |
| Diminution of spatial memory deficit | + | + |
| Increased expression of TrkA in astrocytes | + | + |
| Diminution of Abeta oligomers in microglia | + | - |
| Diminution of Abeta oligomers in astrocytes | + | + |
| Diminution of non-oligomeric Abeta in microglia | + | + |
| Diminution of non-oligomeric Abeta in astrocytes | + | - |

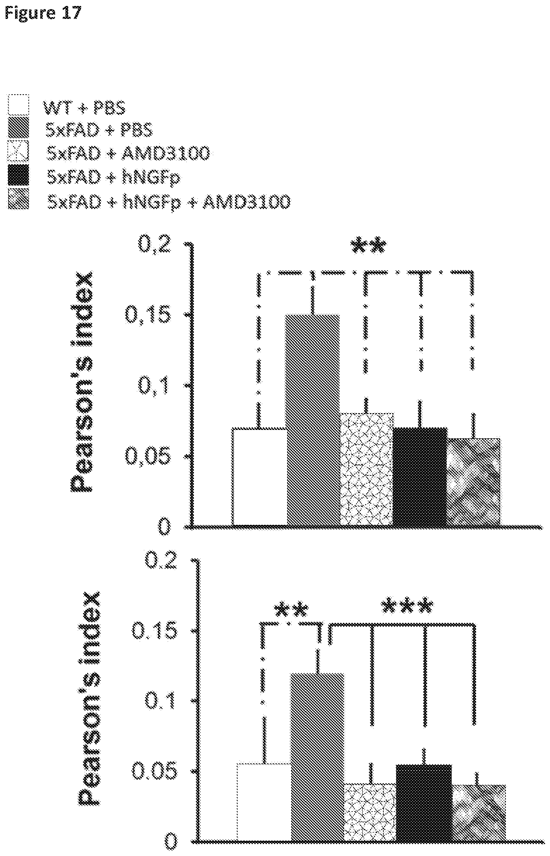

Indeed, the reduction of the immunoreactivity for the Abeta oligomers in the microglia and of the immunoreactivity for non-oligomer forms of Abeta in the astrocytes, by the hNGFP, is not affected by the simultaneous administration of the inhibitor AMD3100 (Fig. 17).

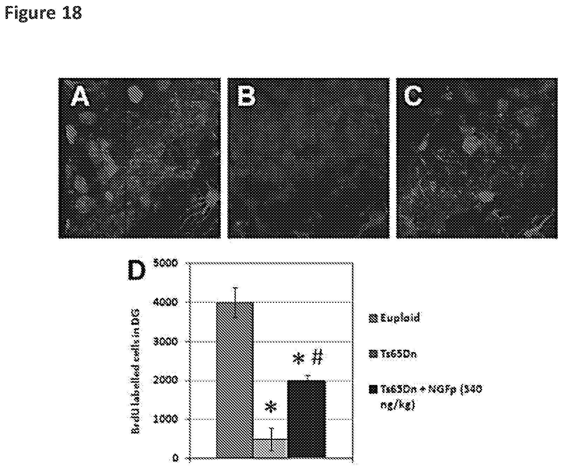

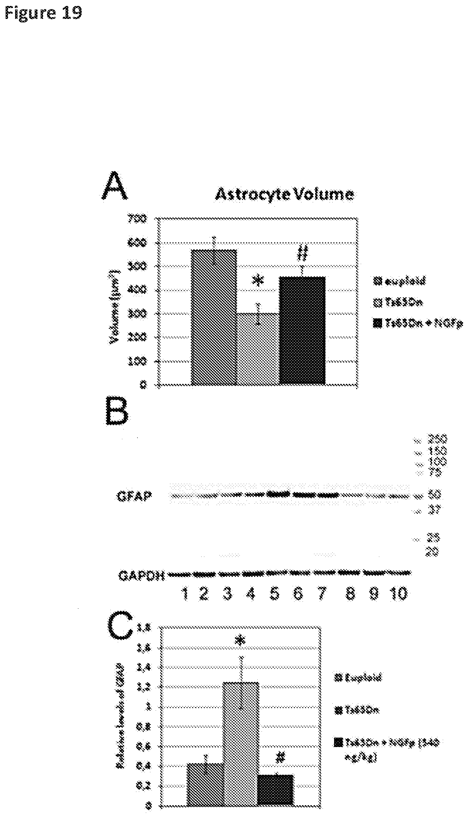

This demonstrates that hNGFp is an activator of SDF-1alpha and that it selectively activates most, but not all, the pathways activated by this chemokine, showing - in a manner that absolutely could not have been predicted beforehand - a more favorable action spectrum. The anti-amyloidogenic effect of hNGFp must necessarily be mediated by the cells of the glia and not by the cholinergic neurons. Microglia and astrocytes are thus unexpectedly a new target of hNGFp, selectively mediated by the chemokine SDF-1alpha. This extends the possibility of therapeutic use thereof to other clinical indications. As evidence thereof, the effectiveness of hNGFp was verified in improving some deficits observed in the model of the Down syndrome, Ts65Dn (Wisniewski et al, 1984; Colombo et al., 2005). Among these, deficits of neurogenesis and alteration of the morphology of the astrocytes. Following intranasal treatment of hNGFp in the Ts65Dn mice, a rescue of the neurogenesis deficits was verified (Fig. 18) along with a return of the volume of the astrocytes by hNGFp to the control levels (Fig. 19), similar to that observed in the model AD, and an accompanying reduction of the expression of the fibrillary acidic protein (GFAP) (Fig. 19).

The Ts65Dn mice also have other aspects of the Down syndrome pathology, including learning and memory deficits and many other typical signs of Alzheimer's neuropathology, including an increase of the peptide Ab, starting from the age of 6 months. It is therefore of interest to verify if the treatment with hNGFp is also capable of improving these aspects of neurodegeneration in the Ts65Dn mice. Ts65Dn mice of 6 months age were treated for three weeks with the same dose of hNGFp, and the expression of the peptide Ab was observed via immunohistochemistry with the antibody 4G8 anti Ab, demonstrating a considerable reduction of the expression of this peptide in the brain. The levels of the peptide Ab, after the intranasal treatment with hNGFp, were measured in the brain of the Ts65Dn mice, by means of ELISA and Western blot, also demonstrating in this case a considerable reduction of the peptide Ab. At the behavioral level, the reduction of the peptide Ab correlates with the observation of a recovery of the behavioral memory deficit, present in these mice, measured with the Y maze protocol. In conclusion, the intranasal administration of hNGFp to Ts65Dn mice significantly improves numerous aspects of the deficits observed in these mice, and of the neuropathology of Alzheimer type, characteristic of Down syndrome.

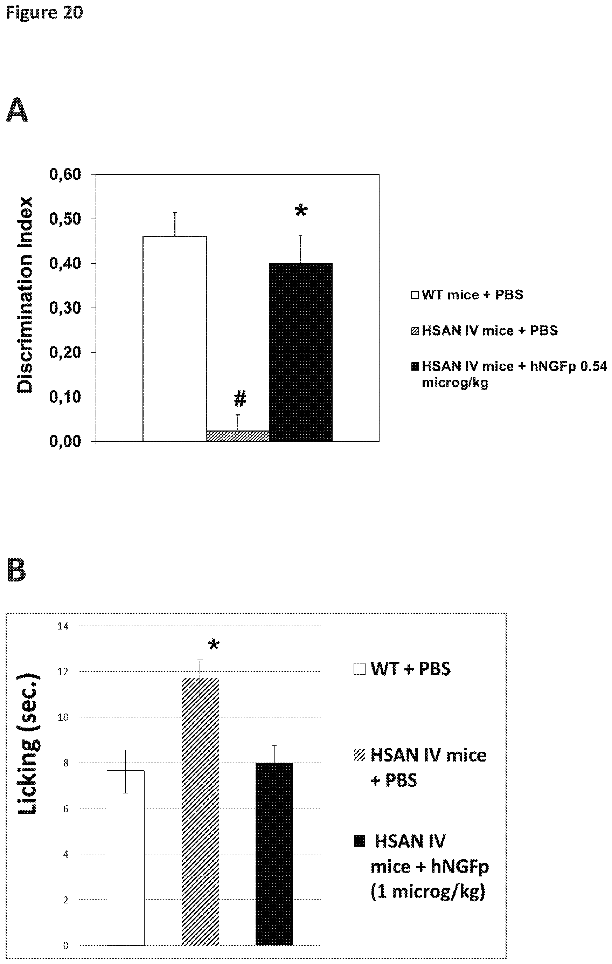

The most generalized neuroprotective effect of hNGFp is also shown in animal models of a rare human disease, the sensory and autonomic neuropathy type IV (also termed HSAN IV). This rare disease is due to mutations of the gene that encodes the receptor TrkA (Indo et al, 2014). The mutations HSAN IV render the receptor TrkA hypofunctional and determine a decreased sensitivity to painful stimuli and learning and memory deficits. The functionality of the receptor p75NTR is however unaltered in such pathology. The supplementation with hNGFp, used in place of the hwild type NGF as preferred agonist of the receptor TrkA, with respect to p75NTR, without however activating the signaling through the receptor p75NTR in an undesired manner, determined: 1) after intranasal administration for a month, the recovery of the cognitive deficits, as demonstrated by the object discrimination test in Fig. 20 A, and 2) the recovery of pain sensitivity, as demonstrated by the hot plate test in Fig. 20 B.

In light of the aforesaid experimental data, pharmaceutical compositions were prepared comprising hNGFp and excipients and/or carriers pharmaceutically acceptable for use in the aforesaid treatments were prepared and in particular: for use in a therapeutic treatment method for said diseases, Down syndrome and sensory and autonomic neuropathy type IV (also termed HSAN IV), which are such to allow a diffused biodistribution of said composition at the nervous system level such that the glia and microglia cells can mediate the anti-amyloidogenic action of hNGFp. Such pharmaceutical compositions can be administered, by way of a non-limiting example, intranasally.

### Bibliography

Barouch R, Kazimirsky G, Appel E, Brodie C. (2001) Nerve growth factor regulates TNF-alpha production in mouse macrophages via MAP kinase activation. J Leukoc Biol. 2001 Jun;69(6):1019-26.
Bezzi P, Domercq M, Brambilla L, Galli R, Schols D, De Clercq E, Vescovi A, Bagetta G, Kollias G, Meldolesi J, Volterra A (2001) CXCR4-activated astrocyte glutamate release via TNFalpha: amplification by microglia triggers neurotoxicity. Nature neuroscience 4:702-710.
Bleul CC, Fuhlbrigge RC, Casasnovas JM, Aiuti A, Springer TA (1996) A highly efficacious lymphocyte chemoattractant, stromal cell-derived factor 1 (SDF-1). J Exp Med 184:1101-1109.
Bockstal V, Geurts N, Magez S (2011) Acute Disruption of Bone Marrow B Lymphopoiesis and Apoptosis of Transitional and Marginal Zone B Cells in the Spleen following a Blood-Stage Plasmodium chabaudi Infection in Mice. J Parasitol Res 2011:534697.
Calì C, Bezzi P. (2010) CXCR4-mediated glutamate exocytosis from astrocytes. J Neuroimmunol. 2010 Jul 27;224(1-2):13-21. doi: 10.1016/j.jneuroim.2010.05.004. Epub 2010 Jun 30.
Colombo J.A., H.D. Reisin, M. Jones, C. Bentham, Development of interlaminar astroglial processes in the cerebral cortex of control and Down's syndrome human cases, Exp Neurol 193 (2005) 207-217.
Covaceuszach S, Capsoni S, Marinelli S, Pavone F, Ceci M, Ugolini G, Vignone D, Amato G, Paoletti F, Lamba D, Cattaneo A (2010) In vitro receptor binding properties of a "painless" NGF mutein, linked to hereditary sensory autonomic neuropathy type V. Biochemical and biophysical research communications 391:824-829.
Covaceuszach S, Capsoni S, Ugolini G, Spirito F, Vignone D, Cattaneo A (2009) Development of a non invasive NGF-based therapy for Alzheimer's disease. Curr Alzheimer Res 6:158-170.
Einarsdottir E, Carlsson A, Minde J, Toolanen G, Svensson O, Solders G, Holmgren G, Holmberg D, Holmberg M (2004) A mutation in the nerve growth factor beta gene (NGFB) causes loss of pain perception. Human molecular genetics 13:799-805.
Frank-Cannon TC, Alto LT, F McAlpine FE and Tansey MG (2009) Does neuroinflammation fan the flame in neurodegenerative diseases? Molecular Neurodegeneration 2009, 4:47 doi:10.1186/1750-1326-4-47.
Guyon A (2014) CXCL12 chemokine and its receptors as major players in the interactions between immune and nervous systems. Frontiers in cellular neuroscience 8:65.
Habbas S, Santello M, Becker D, Stubbe H, Zappia G, Liaudet N, Klaus FR, Kollias G, Fontana A, Pryce CR, Suter T and Volterra A (2015) Neuroinflammatory TNFalpha impairs memory via astrocyte signaling. Cell 163, 1730-1741.
Imitola J, Raddassi K, Park KI, Mueller FJ, Nieto M, Teng YD, Frenkel D, Li J, Sidman RL, Walsh CA, Snyder EY, Khoury SJ (2004) Directed migration of neural stem cells to sites of CNS injury by the stromal cell-derived factor 1alpha/CXC chemokine receptor 4 pathway. Proceedings of the National Academy of Sciences of the United States of America 101:18117-18122.
Indo Y (2014) Neurobiology of pain, interoception and emotional response: lessons from nerve growth factor-dependent neurons. Eur J Neurosci. 39:375-91.
Latta CH, Brothers HM, Wilcock DM (2015) Neuroinflammation in Alzheimer's disease; A source of heterogeneity and target for personalized therapy. Neuroscience 302:103-111.
Malerba F, Paoletti F, Bruni Ercole B, Materazzi S, Nassini R, Coppi E, Patacchini R, Capsoni S, Lamba D, Cattaneo A (2015) Functional Characterization of Human ProNGF and NGF Mutants: Identification of NGF P61SR100E as a "Painless" Lead Investigational Candidate for Therapeutic Applications. PLoS ONE 10:e0136425.
Nagahara AH, Bernot T, Moseanko R, Brignolo L, Blesch A, Conner JM, Ramirez A, Gasmi M, Tuszynski MH (2009) Long-term reversal of cholinergic neuronal decline in aged non-human primates by lentiviral NGF gene delivery. Experimental neurology 215:153-159.
Nayak D, Roth TL, and McGavern DB (2014) Microglia Development and Function. Annual Review of Immunology Vol. 32: 367-402 (Volume publication date March 2014) DOI: 10.1146/annurev-immunol-032713-120240.
Oakley H, Cole SL, Logan S, Maus E, Shao P, Craft J, Guillozet-Bongaarts A, Ohno M, Disterhoft J, Van Eldik L, Berry R, Vassar R (2006) Intraneuronal beta-amyloid aggregates, neurodegeneration, and neuron loss in transgenic mice with five familial Alzheimer's disease mutations: potential factors in amyloid plaque formation. J Neurosci 26:10129-10140.
Parachikova A, Cotman CW (2007) Reduced CXCL12/CXCR4 results in impaired learning and is downregulated in a mouse model of Alzheimer disease. Neurobiol Dis 28:143-153.
Patel JR, McCandless EE, Dorsey D, Klein RS (2010) CXCR4 promotes differentiation of oligodendrocyte progenitors and remyelination. Proceedings of the National Academy of Sciences of the United States of America 107:11062-11067.
Petty JM, Sueblinvong V, Lenox CC, Jones CC, Cosgrove GP, Cool CD, Rai PR, Brown KK, Weiss DJ, Poynter ME, Suratt BT (2007) Pulmonary stromal-derived factor-1 expression and effect on neutrophil recruitment during acute lung injury. J Immunol 178:8148-8157.
Raman D, Milatovic SZ, Milatovic D, Splittgerber R, Fan GH, Richmond A (2011) Chemokines, macrophage inflammatory protein-2 and stromal cell-derived factor-1alpha, suppress amyloid beta-induced neurotoxicity. Toxicol Appl Pharmacol 256:300-313.
Rosenberg MB, Friedmann T, Robertson RC, Tuszynski M, Wolff JA, Breakefield XO, Gage FH (1988) Grafting genetically modified cells to the damaged brain: restorative effects of NGF expression. Science 242:1575-1578.
Salehi A, Delcroix JD, Belichenko PV, Zhan K, Wu C, Valletta JS, Takimoto-Kimura R, Kleschevnikov AM, Sambamurti K, Chung PP, Xia W, Villar A, Campbell WA, Kulnane LS, Nixon RA, Lamb BT, Epstein CJ, Stokin GB, Goldstein LS, Mobley WC (2006) Increased App expression in a mouse model of Down's syndrome disrupts NGF transport and causes cholinergic neuron degeneration. Neuron 51:29-42.
Santello, M., and Volterra, A. (2012). TNFa in synaptic function: switching gears. Trends Neurosci. 35, 638-647.
Smith DE, Roberts J, Gage FH, Tuszynski MH (1999) Age-associated neuronal atrophy occurs in the primate brain and is reversible by growth factor gene therapy. Proc Natl Acad Sci U S A 96:10893-10898.
Stumm R, Kolodziej A, Schulz S, Kohtz JD, Hollt V (2007) Patterns of SDF-1alpha and SDF-1gamma mRNAs, migration pathways, and phenotypes of CXCR4-expressing neurons in the developing rat telencephalon. The Journal of comparative neurology 502:382-399.
Takei Y, Laskey R. (2008) Interpreting crosstalk between TNF-alpha and NGF: potential implications for disease. Trends Mol Med. 2008 Sep;14(9):381-8. doi: 10.1016/j.molmed.2008.07.002. Epub 2008 Aug 6.
Tuszynski MH (2000) Intraparenchymal NGF infusions rescue degenerating cholinergic neurons. Cell Transplant 9:629-636.
Wang Y, Deng Y, Zhou GQ (2008) SDF-1alpha/CXCR4-mediated migration of systemically transplanted bone marrow stromal cells towards ischemic brain lesion in a rat model. Brain research 1195:104-112.
Wisniewski KE, Laure-Kamionowska M, Wisniewski HM Evidence of arrest of neurogenesis and synaptogenesis in brains of patients with Down's syndrome. N Engl J Med. 1984 Nov 1;311(18):1187-8.
Zhang C, Wang ZJ, Lok KH, Yin M (2012) beta-amyloid42 induces desensitization of CXC chemokine receptor-4 via formyl peptide receptor in neural stem/progenitor cells. Biol Pharm Bull 35:131-138.
Zhang Q, Guo R, Schwarz EM, Boyce BF, Xing L (2008) TNF inhibits production of stromal cell-derived factor 1 by bone stromal cells and increases osteoclast precursor mobilization from bone marrow to peripheral blood. Arthritis Res Ther 10:R37.

## Claims

1. A Human mutated form of NGF comprising two mutations, a first mutation being represented by the substitution of the proline amino acid in position 61 with a serine, a second mutation being represented by the substitution of an amino acid in one of the positions 95-101, for use in a treatment method for Down syndrome, wherein the second mutation regards the substitution of the arginine amino acid in position 100 with an amino acid selected in the group constituted by tryptophan and glutamic acid said human mutated form of NGF being capable to act as agent for the activation of the chemokine SDF-1alpha, MIP-1alpha, MIP-1gamma and as agent for the inhibition of the activity of the cytokine TNF alpha.

2. A Human mutated form of NGF comprising two mutations, a first mutation being represented by the substitution of the proline amino acid in position 61 with a serine, a second mutation being represented by the substitution of an amino acid in one of the positions 95-101, for use in a treatment method for Sensory and Autonomic Neuropathy type IV, wherein the second mutation regards the substitution of the arginine amino acid in position 100 with an amino acid selected in the group constituted by tryptophan and glutamic acid, said human mutated form of NGF being capable to act as agent for the activation of the chemokines SDF-1alpha, MIP-1alpha, MIP-1gamma and as agent for the inhibition of the activity of the cytokine TNF alpha.

## Patentansprüche

1. Menschliche mutierte Form von NGF, die zwei Mutationen aufweist, wobei eine erste Mutation durch die Substitution der Prolin-Aminosäure in Position 61 durch ein Serin dargestellt wird, eine zweite Mutation durch die Substitution einer Aminosäure in einer der Positionen 95-101 dargestellt wird, zur Verwendung in einem Behandlungsverfahren für das Down-Syndrom, wobei die zweite Mutation die Substitution der Arginin-Aminosäure in Position 100 durch eine Aminosäure, die aus der durch Tryptophan und Glutaminsäure gebildeten Gruppe ausgewählt ist, betrifft, wobei die menschliche mutierte Form von NGF in der Lage ist, als Mittel zur Aktivierung der Chemokine SDF-1alpha, MIP-1alpha, MIP-1gamma und als Mittel zur Hemmung der Aktivität des Zytokins TNF-alpha zu wirken.

2. Menschliche mutierte Form von NGF, die zwei Mutationen aufweist, wobei eine erste Mutation durch die Substitution der Prolin-Aminosäure in Position 61 durch ein Serin dargestellt wird, eine zweite Mutation durch die Substitution einer Aminosäure in einer der Positionen 95-101 dargestellt wird, zur Verwendung in einem Behandlungsverfahren für sensorische und autonome Neuropathie Typ IV, wobei die zweite Mutation die Substitution der Arginin-Aminosäure in Position 100 durch eine Aminosäure, die aus der durch Tryptophan und Glutaminsäure gebildeten Gruppe ausgewählt ist, betrifft, wobei die menschliche mutierte Form von NGF in der Lage ist, als Mittel zur Aktivierung der Chemokine SDF-1alpha, MIP-1alpha, MIP-1gamma und als Mittel zur Hemmung der Aktivität des Zytokins TNF-alpha zu wirken.

## Revendications

1. Forme mutée humaine de NGF comprenant deux mutations, une première mutation étant représentée par le remplacement de l'acide aminé proline à la position 61 par une sérine, une deuxième mutation étant représentée par le remplacement d'un acide aminé à l'une des positions 95 à 101, pour une utilisation dans une méthode de traitement du syndrome de Down, dans laquelle la deuxième mutation concerne le remplacement de l'acide aminé arginine à la position 100 par un acide aminé choisi dans le groupe constitué par le tryptophane et l'acide glutamique, ladite forme mutée humaine de NGF étant capable d'agir comme un agent pour l'activation des chimiokines SDF-1alpha, MIP-1alpha, MIP-1gamma et en tant qu'agent pour l'inhibition de l'activité de la cytokine TNF alpha.

2. Forme mutée humaine de NGF comprenant deux mutations, une première mutation étant représentée par le remplacement de l'acide aminé proline à la position 61 par une sérine, une deuxième mutation étant représentée par le remplacement d'un acide aminé à l'une des positions 95 à 101, pour une utilisation dans une méthode de traitement de la neuropathie sensitive et autonome de type IV, dans laquelle la deuxième mutation concerne le remplacement de l'acide aminé arginine à la position 100 par un acide aminé choisi dans le groupe constitué par le tryptophane et l'acide glutamique, ladite forme mutée humaine de NGF étant capable d'agir comme un agent pour l'activation des chimiokines SDF-1alpha, MIP-1alpha, MIP-1gamma et en tant qu'agent pour l'inhibition de l'activité de la cytokine TNF alpha.
